Europäisches Patentamt

European Patent Office

Office européen des brevets

(11) Publication number: **0 050 867**
**B1**

# EUROPEAN PATENT SPECIFICATION

(45) Date of publication of patent specification: **13.01.88**

(21) Application number: **81108872.3**

(22) Date of filing: **24.10.81**

(51) Int. Cl.⁴: **A 61 M 31/00,** A 61 F 5/47, A 61 K 9/02

(54) **Vaginal ring.**

(30) Priority: **28.10.80 DE 3040978**

(43) Date of publication of application:
**05.05.82 Bulletin 82/18**

(45) Publication of the grant of the patent:
**13.01.88 Bulletin 88/02**

(84) Designated Contracting States:
**AT BE CH DE FR GB IT LI LU NL SE**

(56) References cited:
**FR-A-2 247 398**
**FR-A-2 270 899**
**GB-A-1 264 731**
**US-A-3 279 996**
**US-A-3 545 439**
**US-A-3 845 761**
**US-A-3 854 480**
**US-A-4 012 496**
**US-A-4 215 691**

(73) Proprietor: **SCHERING AKTIENGESELLSCHAFT**
**Berlin und Bergkamen**
**Müllerstrasse 170/178 Postfach 65 03 11**
**D-1000 Berlin 65 (DE)**

(72) Inventor: **Zimmermann, Ingfried, Dr.**
**Gollanczstrasse 28c**
**D-1000 Berlin 28 (DE)**
Inventor: **Windt, Fred**
**Steglitzer Damm 116a**
**D-1000 Berlin 41 (DE)**
Inventor: **Reck, Hans-Jürgen**
**Wilhelmstrasse 167**
**D-1000 Berlin 20 (DE)**

(74) Representative: **Coulson, Antony John et al**
**ABEL & IMRAY Northumberland House 303-306**
**High Holborn**
**London, WC1V 7LH (GB)**

Courier Press, Leamington Spa, England.

## Description

The present invention relates to a vaginal ring of the type defined in the pre-characterizing portion of appended claim 1.

Vaginal rings composed of synthetic resins, and containing active substances, have been known for some time. DE—A—2 450 107 and its U.S. equivalent US—A—4 012 496 describe a vaginal ring consisting of a supporting ring with one or two continuous pocket-like indentations adapted to accommodate active-substance-containing rings which fit into these indentations and are made of a synthetic low temperature vulcanisable (LTV) silicone elastomer resin.

U.S. Patent 3 920 805 likewise describes a vaginal ring of a silicone elastomer consisting of a core free of active substance and a coating containing an active substance.

In use of the vaginal rings described in U.S. Patents Nos. 4 012 496 and 3 920 805 the active substance is released directly to the surrounding body parts from the ring or coating containing the active substance. The rings have the disadvantage that the rate of release of active substance is not constant over the desired time period. Instead, there is an initial surge of active substance, so that the user of the device receives an undesirably large amount of the active substance, after which the amount released per unit time becomes increasingly smaller.

U.S. Patent No. 3 854 480 relates to a drug-delivery system for releasing a drug at a controlled rate for a prolonged period of time. The system is formed from an inner core having solid particles of drug dispersed therethrough, the inner core being surrounded by a relatively thick outer polymeric membrane through which the drug can diffuse. One embodiment of the system consists of a silicone elastomer containing the active agent, surrounded by a polyethylene hose having a wall thickness of about 0.4 mm. It is stated in this patent that the system can release drugs at a rate which does not vary with time, and that the system can be used for any drug capable of diffusing through a polymeric membrane at a therapeutically effective rate. It is found in practice, however, that for certain important contraceptive agents with which the present invention is concerned, the disclosed system does not provide uniform release of the active substances in a sufficient quantity and over a sufficient period.

U.S. Patent No. 3 545 439, which is referred to in U.S. Patent No. 3 920 805 discussed above, discloses a vaginal ring comprising a ring of drug-permeable polymeric material, for example, polysiloxanes curable on heating or room-temperature curable polysiloxanes. Medication for release to a patient can be introduced into hollow cavities in the ring, or directly introduced into the plastics material itself. As in the case of the patents discussed above, however, the rings disclosed in U.S. Patent No. 3 545 439 do not exhibit constant release of the active substance and, in particular, are not free from the problem of an initial surge in the release of active substance discussed above.

The problem to be solved by the invention was the provision of a vaginal ring from which a pharmacologically active substance, chosen from a specific and important group of contraceptive agents, could be released over a prolonged period of time (several monthly cycles) with uniform regularity, in sufficient quantity, and without overdosage of the active substance at the beginning of the treatment.

In accordance with the invention the above problem is solved by the provision of a vaginal ring comprising a physiologically acceptable supporting ring and a first, continuously encircling, physiologically acceptable layer on the outer rim of the supporting ring, the first layer incorporating a pharmacologically active substance and the supporting ring being essentially free of pharmacologically active substance characterised in that the supporting ring comprises a low temperature vulcanisable silicone elastomer comprising

80—96.5% by weight polydimethylvinylsiloxane
    1—10% by weight of tetramethyltetravinylcyclotetrasiloxane
    2—20% by weight of polydimethyl hydrogen siloxane
    10—100 ppm of platinum catalyst, and, optionally,
    0.5—10% by weight of highly disperse silicic acid, the first layer comprises a low temperature vulcanisable silicone elastomer comprising
    75—97% by weight polydimethylvinylsiloxane
    1—10% by weight of tetramethyltetravinylcyclotetrasiloxane
    1—10% by weight of polydimethyl hydrogen siloxane
    10—100 ppm of platinum catalyst and
    1—10% by weight of active substance,

the active substance is selected from ethynylestradiol, ethisterone, norethisterone acetate, norethynodrel, levonorgestrel and gestoden, and mixtures thereof,

the ring also comprises a second physiologically tolerable layer, not derived from the first layer, coating the first layer,

the second layer being essentially free of pharmacologically active substance but being permeable to the pharmacologically active substance in the first layer, and

the second layer comprising a low temperature vulcanisable silicone elastomer comprising
    75—97% by weight of polydimethylvinylsiloxane
    1—10% by weight of tetramethyltetravinylcyclotetrasiloxane
    2—20% by weight of polydimethyl hydrogen siloxane, and

10—100 ppm of platinum catalyst,

the ratio of the thickness of the first layer to that of the second layer being about 5—50:1.

The second layer in the vaginal ring of the invention is not derived from the first layer, and advantageously comprises a different material from that from which the first layer is formed.

The present invention makes it possible to provide a vaginal ring having a release of active substance which is of uniform regularity, of sufficient quantity and of long duration.

The supporting ring, which is essentially free of active substance, can have a continuous groove to accommodate the first layer.

In the accompanying drawings, in which like reference characters designate the same or similar parts throughout the several views,

Figure I schematically illustrates a top view of one embodiment of a vaginal ring of this invention;

Figures II and III show a section A—B of two embodiments of such a ring; and

Figure IV shows the uniformity of release of active substance from a vaginal ring of this invention. (Release per day over a time period of more than 2 months).

The vaginal ring of this invention has an average outer dimension of 0.5—20 cm, the exact size being dependent on the particular application. In the case of relatively small mammals, such as dogs, the ring size will be smaller than in the case of relatively larger mammals, such as horses or cows. For Rhesus monkeys, for example, the outer diameter is about 2—3 cm. In vaginal rings for human females, the outer diameter is about 5—7 cm and the largest width of the ring cross section (see, e.g., section A—B of Figures I to III) is 5—10 mm. Dimensional details for any ring for any application can be determined, if necessary, using routine preliminary experiments.

In a preferred embodiment of the supporting ring 1, a groove is contained in its outer rim for engaging layer 2. (See, e.g., Figure III as well as other variants shown in U.S. Patent 4 012 496).

U.S. Patent 4 012 496 and DE—A—26 16 064 may be referred to, where appropriate, in connection with overall sizes and shapes, cross-sectional sizes and shapes of the ring and individual ring components, methods of construction and preparation of each ring component.

A special embodiment of the invention has an outer diameter of 58 to 60 mm, an inner diameter of 50 to 52 mm, and the largest width of the non-circular cross-section (see, e.g., section A—B of Fig. II or III) is 6.5 to 7.5 mm.

The supporting ring 1 comprises a physiologically acceptable low temperature vulcanisable (LTV) silicone elastomer whose vulcanisation behaviour with respect to the layer 2 containing the active substance does not cause any technical problems. The LTV silicone elastomer has the following composition:

80—96.5% by weight of polydimethylvinylsiloxane

1—10% by weight of tetramethyltetravinylcyclotetrasiloxane

2—20% by weight of polydimethyl hydrogen siloxane

10—100 ppm of platinum catalyst, and, optionally,

0.5—10% by weight of highly disperse silicic acid.

Suitable platinum catalysts include metallic platinum *per se*, platinum on support materials, such as silica gel, or platinum in the form of its salts, e.g., platinum carbonyl dichloride or platinum dicarbonyl dichloride, or as hexachloroplatinic acid. Also suitable are platinum complexes of unsaturated siloxanes (cf. U.S. Patent No. 4 166 078).

The composition for producing the supporting ring can optionally also contain, in addition to the optional highly dispersed silicic acid, which influences primarily only the mechanical properties, also other auxiliary materials, such as, for example, tensides, solubilisers and colouring agents, as long as they are inert with respect to the total system.

The layer 2 containing the active substance comprises as a base composition, a physiologically acceptable LTV silicone elastomer from which the substance can be released, e.g., as described in U.S. Patent 4 012 496. The LTV silicone elastomer has the following composition:

75—97% by weight polydimethylvinylsiloxane

1—10% by weight of tetramethyltetravinylcyclotetrasiloxane

1—10% by weight of polydimethyl hydrogen siloxane

10—100 ppm of platinum catalyst and

1—10% by weight of active substance.

The active substances that may be used in the ring of the invention are nonionic, lipid-soluble, steroid hormones having a progestational or estrogenic activity. The active substance(s) are selected from ethynylestradiol, ethisterone, norethisterone acetate, norethynodrel, levonorgestrel, and gestoden.

The second layer 3, free of active substance, comprises LTV silicone elastomer having the following composition:

75—97% by weight of polydimethylvinylsiloxane

1—10% by weight of tetramethyltetravinylcyclotetrasiloxane

2—20% by weight of polydimethyl hydrogen siloxane and

10—100 ppm of platinum catalyst.

The rings of this invention may be manufactured by any suitable method, preferably by the injection molding technique for layers 1 and 2.

3

Specific details for forming any component or overall device of this invention or for forming any composition can be determined readily from U.S. Patent 4 012 496, for example. All starting materials are known.

For example, the amount of polydimethylvinylsiloxane employed is advantageously first divided typically into portions of 1 to 1. One portion is used together with the polydimethyl hydrogen siloxane, and the other together with the platinum catalyst. The two portions are combined only shortly before vulcanisation and are crosslinked by temperature increase.

The active substance is suitably micronised before being incorporated into the liquid mixture of the starting components.

The layer 3, free of active ingredient, may likewise be applied by any suitable method, such as dipping or spraying. The layer thickness is advantageously 10—1,000, preferably 100—500 µm. The ratio of thickness of layer 2 to layer 3 is 5—50:1, preferably 20—40:1.

The vaginal ring of this invention has the advantage that the active agent is released over a relatively long period of time within the limits of the dosage required for the biological effect desired, e.g., the dose necessary for inhibition of ovulation, in a regular and uniform fashion.

The use of the vaginal rings of this invention is fully conventional including methods and precautions of insertion, maintenance and removal, except of course that advantage can be taken of the long-term and regular drug release provided.

The following Examples illustrate the invention. In the Examples, all temperatures are given uncorrected in degrees Celsius; unless otherwise indicated, all parts and percentages are by weight.

Example 1

The following components are thoroughly mixed for producing the supporting ring 1
232.577 g of vinyl-terminated polydimethylsiloxane having an average molecular weight of $\overline{M}$ 5854,
17.423 g of tetramethyltetravinylcyclotetrasiloxane and
25.000 mg of platinum (mixture A1), as well as
193.789 g of vinyl-terminated polydimethylsiloxane and
56.211 g of polydimethylhydrogen siloxane (mixture B1)

The two mixtures A1 and B1 are homogenised in a static mixing pipe, introduced into an injection molding tool, and vulcanised during a period of 60 seconds at 100°C into rings having a cross section of a circular segment.

The Shore A hardness is 45, and the elongation at rupture is 50%.

To produce the layer 2, containing the active substance, the following components are mixed intensively.
49.86 g of vinyl-terminated polydimethylsiloxane ($\overline{M}$=8170),
0.14 g of tetramethyltetravinylcyclotetrasiloxane,
5.00 mg of platinum, and
1.814 g of levonorgestrel (mixture A2), as well as
48.61 g of vinyl-terminated polydimethylsiloxane ($\overline{M}$=8170),
1.39 g of polydimethyl hydrogen siloxane, and
1.814 g of levonorgestrel (Mixture B2).

The two mixtures A2 and B2 are homogenised in a static mixing pipe, introduced into an injection molding tool with inserted supporting rings 1, and vulcanised onto the outer rim of the supporting ring 1 for a period of 60 seconds at 100°C.

The thus-produced vaginal rings are sprayed, in the region of the layer 2 containing the active substance with a layer 3 of silicone elastomer having a thickness of about 200 µm. This layer forms a composite vulcanisate with the layer 2 disposed therebelow.

The silicone elastomer mixture for the layer 3 free of active substance is composed of
42.53 g of vinyl-terminated polydimethylsiloxane ($\overline{M}$ 4561),
5.72 g of polydimethyl hydrogen siloxane,
1.75 g of tetramethyltetravinylcyclotetrasiloxane, and
50 ppm of platinum.

The individual ring contains accordingly 0.8% of active substance and has a release rate of 70.5 µg/d.

Example 2

Analogously to Example 1, supporting rings 1 are produced from
233.98 g of vinyl-terminated polydimethylsiloxane ($\overline{M}$ 8170),
16.02 g of tetramethyltetravinylcyclotetrasiloxane,
25.00 mg of platinum, and
13.158 g of highly disperse silicic acid, as well as
203.92 g of vinyl-terminated polydimethylsiloxane ($\overline{M}$=8170),
46.08 g of polydimethyl hydrogen siloxane, and
13.158 g of highly disperse silicic acid.

The Shore A hardness is 65, and the elongation at rupture is 75%.

4

A layer 2 containing active substance is vulcanised onto the supporting rings 1, this layer having the following composition:

239.38 g of vinyl-terminated polydimethylsiloxane ($\overline{M}$=8170),

10.62 g of tetramethyltetravinylcyclotetrasiloxane,

25.00 mg of platinum catalyst,

27.778 g of levonorgestrel, as well as

217.70 g of vinyl-terminated polydimethylsiloxane ($\overline{M}$=8170),

32.30 g of polydimethyl hydrogen siloxane,

27.778 g of levonorgestrel.

Thereafter a layer 3 free of active substance and having a thickness of 350 µm is applied to the layer 2 containing the active ingredient, this layer 3 having the following composition:

47.061 g of vinyl-terminated polydimethylsiloxane ($\overline{M}$ 8170),

0.681 g of tetramethyltetravinylcyclotetrasiloxane,

2.258 g of polydimethyl hydrogen siloxane, and

50 ppm of platinum.

The individual ring contains accordingly 2.3% by weight of active substance and has a release rate of 45 µg/d.

Example 3

The same blend as set forth in Example 1 is used for producing a supporting ring 1, consisting of polydimethylsiloxane, tetramethyltetravinylcyclotetrasiloxane, platinum, as well as polydimethyl hydrogen siloxane.

The two mixtures A1 and B1 are homogenised in a static mixing pipe, introduced into an injection molding tool, and vulcanised for a period of 60 seconds at 100°C into rings having a continuous groove, as shown in Figure III.

Subsequently, a composition containing an active substance, consisting of the two mixtures A2 and B2, Example 1, is injected into the groove and vulcanised for 60 seconds at 100°C. Thereafter, the layer 3, free of active substance and having the same composition as in Example 1, is applied to the thus-manufactured ring.

The individual ring has an active agent release rate of 20 µg/d.

Example 4

The in vitro release rates (see Fig. IV) are measured as follows:

The ring is fitted to a support and placed in vessels containing 200 ml of double-distilled water. The ring is fixed about 10 mm above the bottom of the vessel which has a diameter of 85 mm and a height of 90 mm. After an incubation period of 24 hours at 37°C the levonorgestrel-containing water is exchanged and analysed. The levonorgestrel dissolved in the water is concentrated by means of a chromatographic column filled with lipophilised silica gel. By pressing the water through the column the steroid is bound by the aliphatic residues to the surface of the silica gel. The bound steroid is then removed by elution of the column with 5 ml of methanol. The concentration of steroid is determined spectrophotometrically at a wavelength of 248 nm.

The preceding Examples can be repeated with similar success by substituting the generically or specifically described reactants and/or operating conditions of this invention for those used in the preceding Examples.

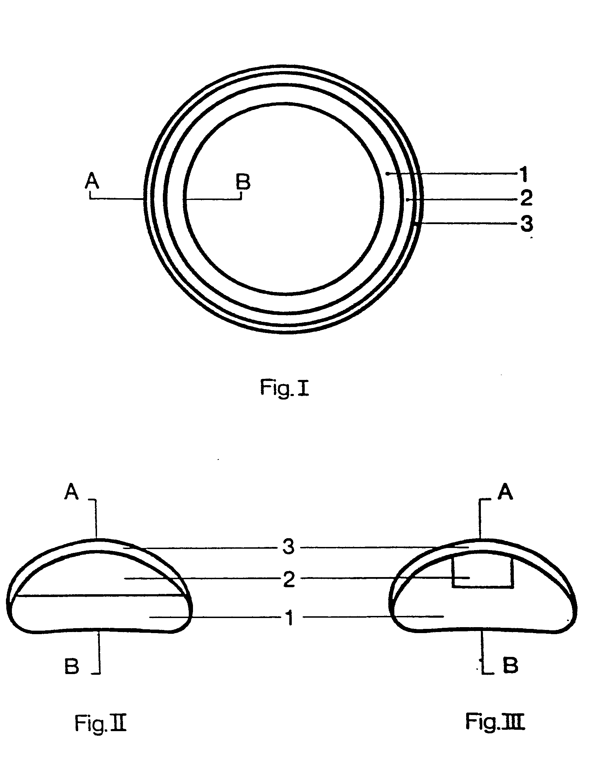

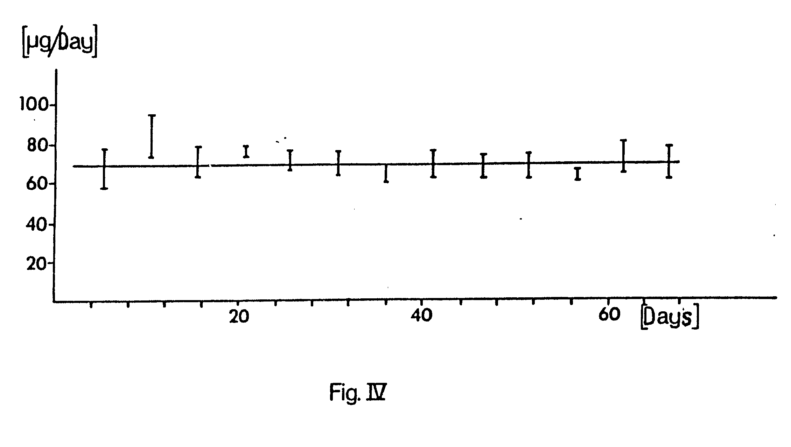

**Claims**

1. A vaginal ring which comprises a physiologically acceptable supporting ring and a first, continuously encircling, physiologically acceptable layer on the outer rim of the supporting ring, the first layer incorporating a pharmacologically active substance and the supporting ring being essentially free of pharmacologically active substance characterised in that

the supporting ring comprises a low temperature vulcanisable silicone elastomer comprising,

80—96.5% by weight polydimethylvinylsiloxane

1—10% by weight of tetramethyltetravinylcyclotetrasiloxane

2—20% by weight of polydimethyl hydrogen siloxane

10—100 ppm of platinum catalyst, and optionally,

0.5—10% by weight of highly disperse silicic acid,

the first layer comprises a low temperature vulcanisable silicone elastomer comprising

75—97% by weight polydimethylvinylsiloxane

1—10% by weight of tetramethyltetravinylcyclotetrasiloxane

1—10% by weight of polydimethyl hydrogen siloxane

10—100 ppm of platinum catalyst and

1—10% by weight of active substance,

the active substance is selected from ethynylestradiol, ethisterone, norethisterone acetate, norethynodrel, levonorgestrel and gestoden, and mixtures thereof,

the ring also comprises a second physiologically tolerable layer, not derived from the first layer, coating the first layer,

the second layer being essentially free of pharmacologically active substance but being permeable to the pharmacologically active substance in the first layer, and

the second layer comprising a low temperature vulcanisable silicone elastomer comprising

75—97% by weight of polydimethylvinylsiloxane

1—10% by weight of tetramethyltetravinylcyclotetrasiloxane

2—20% by weight of polydimethyl hydrogen siloxane, and

10—100 ppm of platinum catalyst,

the ratio of the thickness of the first layer to that of the second layer being about 5—50:1.

2. A vaginal ring as claimed in claim 1, wherein the thickness of the second layer is 10—1000 µm.

3. A vaginal ring as claimed in claim 1, wherein the thickness of the second layer is 100—500 µm.

4. A vaginal ring as claimed in any one of claims 1 to 3, wherein the silicone elastomer of the second layer is different from that of the first layer.

5. A vaginal ring as claimed in any one of claims 1 to 4, in which the arrangement is such that the active substance is transferred to a part only of the inner surface of the second layer.

6. A vaginal ring as claimed in any one of claims 1 to 4, wherein the supporting ring has a continuous groove in its outer rim which mates with the first layer.

7. A vaginal ring as claimed in any one of claims 1 to 6, wherein the second layer has been applied to the first layer by spraying.

8. A vaginal ring as claimed in any one of claims 1 to 6, wherein the second layer has been applied to the first layer by dipping.

9. A vaginal ring as claimed in any one of claims 1 to 8, wherein the ratio of the thickness of the first layer to that of the second layer is 20—40:1.

**Patentansprüche**

1. Vaginalring mit einem physiologisch verträglichen Stützring und einer ersten zusammenhängend umlaufenden, physiologisch verträglichen Schicht auf dem Außenrand des Stützringes, wobei die erste Schicht einen pharmakologischen Wirkstoff enthält und der Stützring im wesentlichen frei von pharmakologischen Wirkstoffen ist, dadurch gekennzeichnet, daß der Stützring ein bei niedriger Temperatur vulkanisierbares Silikonelastomer mit

80—96,5 Gew.-% Polydimethylvinylsiloxan,

1—10 Gew.-% Tetramethyltetravinylcyclotetrasiloxan,

2—20 Gew.-% Polydimethylhydrogensiloxan,

10—100 ppm eines Platinkatalysators und ggf.

0,5—10 Gew.-% hoch disperser Kieselsäure umfaßt,

wobei die erste Schicht ein bei niedriger Temperatur vulkanisierbares Silikonelastomer mit

75—97 Gew.-% Polydimethylvinylsiloxan,

1—10 Gew.-% Tetramethyltetravinylcyclotetrasiloxan,

1—10 Gew.-% Polydimethylhydrogensiloxan,

10—100 ppm eines Platinkatalysators und

1—10 Gew.-% eines Wirkstoffs umfaßt,

wobei der Wirkstoff aus der aus Ethinylestradiol, Ethisteron, Norethisteronacetat, Norethinodrel, Levonorgestrel, Gestoden und deren Mischungen bestehenden Gruppe ausgewählt ist,

wobei der Ring ferner eine zweite physiologisch verträgliche Schicht umfaßt, die sich nicht von der ersten Schicht ableitet und die die erste Schicht bedeckt,

wobei die zweite Schicht im wesentlichen frei von pharmakologischen Wirkstoffen, jedoch für den pharmakologischen Wirkstoff der ersten Schicht durchlässig ist, und

wobei die zweite Schicht ein bei niedriger Temperatur vulkanisierbares Silikonegastomer mit

75—97 Gew.-% Polydimethylvinylsiloxan,

1—10 Gew.-% Tetramethyltetravinylcyclotetrasiloxan,

2—20 Gew.-% Polydimethylhydrogensiloxan und

10—100 ppm eines Platinkatalysators umfaßt,

wobei das Verhältnis der Stärke der ersten Schicht zur Stärke der zweiten Schicht etwa 5—50:1 beträgt.

2. Vaginalring nach Anspruch 1, bei dem die Stärke der zweiten Schicht 10—1000 µm beträgt.

3. Vaginalring nach Anspruch 1, bei dem die Stärke der zweiten Schicht 100—500 µm beträgt.

4. Vaginalring nach einem der Ansprüche 1 bis 3, bei dem das Silikonelastomer der zweiten Schicht von dem der ersten Schicht verschieden ist.

5. Vaginalring nach einem der Ansprüche 1 bis 4 mit einer derartigen Anordnung, daß der Wirkstoff nur auf einen Bereich der Innenfläche der zweiten Schicht übertragen wird.

6. Vaginalring nach einem der Ansprüche 1 bis 4, bei dem der Stützring in seinem Außenrand eine durchgehende Nut aufweist, die der ersten Schicht angepaßt ist.

7. Vaginalring nach einem der Ansprüche 1 bis 6, bei dem die zweite Schicht auf die erste Schicht durch Sprühen aufgebracht ist.

8. Vaginalring nach einem der Ansprüche 1 bis 6, bei die zweite Schicht auf die erste Schicht durch Eintauchen aufgebracht ist.

9. Vaginalring nach einem der Ansprüche 1 bis 8, bei dem das Verhältnis der Stärke der ersten Schicht zur Stärke der zweiten Schicht 20—40:1 beträgt.

**Revendications**

1. Un anneau intravaginal qui comprend un anneau de support physiologiquement acceptable et une première couche physiologiquement acceptable, formant une ceinture continue, disposée sur le bord externe de l'anneau de support, la première couche contenant une substance pharmacologiquement active et l'anneau de support étant essentiellement dépourvu de substance pharmacologiquement active, caractérisé en ce que l'anneau de support est constitué d'un élastomère de silicone vulcanisable à basse température comprenant

80—96,5% en poids de polydiméthylvinylsiloxane,

1—10% en poids de tétraméthyltétravinylcyclotétrasiloxane,

2—20% en poids de polydiméthylhydrogénosiloxane,

10—100 ppm de catalyseur au platine, et facultativement,

0,5—10% en poids d'acide silicique très dispersé,

la première couche est constituée d'un élastomère de silicone vulcanisable à basse température comprenant

75—97% en poids de polydiméthylvinylsiloxane,

1—10% en poids de tétraméthyltétravinylcyclotétrasiloxane,

1—10% en poids de polydiméthylhydrogénosiloxane,

10—100 ppm de catalyseur au platine, et

1—10% en poids de substance active,

la substance active est choisie parmi l'éthynyloestradiol, l'éthistérone, l'acétate de noréthistérone, le noréthynodrel, le lévonorgestrel et le gestodène, et leurs mélanges,

l'anneau comprend également une seconde couche physiologiquement acceptable, non dérivée de la première couche, revêtant la première couche,

la seconde couche étant essentiellement dépourvue de substance pharmacologiquement active mais étant perméable à la substance pharmacologiquement active contenue dans la première couche, et

la seconde couche étant constituée d'un élastomère de silicone vulcanisable à basse température comprenant

75—97% en poids de polydiméthylvinylsiloxane,

1—10% en poids de tétraméthyltétravinylcyclotétrasiloxane,

2—20% en poids de polydiméthylhydrogénosiloxane, et

10—100 ppm de catalyseur au platine,

le rapport de l'épaisseur de la première couche à celle de la seconde couche étant d'environ 5—50:1.

2. Un anneau intravaginal tel que revendiqué dans la revendication 1, dans lequel l'épaisseur de la seconde couche est de 100—1000 um.

3. Un anneau intravaginal tel que revendiqué dans la revendication 1, dans lequel l'épaisseur de la seconde couche est de 100—500 µm.

4. Un anneau intravaginal tel que revendiqué dans l'une quelconque des revendications 1 à 3, dans lequel l'élastomère de silicone de la seconde couche est différent de celui de la première couche.

5. Un anneau intravaginal tel que revendiqué dans l'une quelconque des revendications 1 à 4, dans lequel l'agencement est tel que la substance active n'est transférée qu'à une partie seulement de la surface interne de la seconde couche.

6. Un anneau intravaginal tel que revendiqué dans l'une quelconque des revendications 1 à 4, dans lequel l'anneau de support présente une gorge continue pratiquée dans son bord externe et qui est adaptée à la première couche.

7. Un anneau intravaginal tel que revendiqué dans l'une quelconque des revendications 1 à 6, dans lequel la seconde couche a été appliquée à la première couche par pulvérisation.

8. Un anneau intravaginal tel que revendiqué dans l'une quelconque des revendications 1 à 6, dans lequel la seconde couche a été appliquée à la première couche par trempage.

9. Un anneau intravaginal tel que revendiqué dans l'une quelconque des revendications 1 à 8, dans lequel le rapport de l'épaisseur de la première couche à celle de la seconde couche est de 20—40:1.

Fig. I

Fig. II

Fig. III

[µg/Day]

100 — 80 — 60 — 40 — 20 —

[Days]

20  40  60

Fig. IV